# EUROPÄISCHE PATENTSCHRIFT

(11) **EP 2 924 285 B1**
(45) Veröffentlichungstag und Bekanntmachung des Hinweises auf die Patenterteilung: **13.12.2017**
(21) Anmeldenummer: 14161813.2
(22) Anmeldetag: 26.03.2014
(51) Int. Cl.: F04B 1/02, F04B 1/04, F04B 53/00

(54) **Sterilisierbare Pumpeinheit**
Sterilisable pump unit
Unité de pompe stérilisable

(43) Veröffentlichungstag der Anmeldung: 30.09.2015
(73) Patentinhaber: Erbe Elektromedizin GmbH, 72072 Tübingen (DE)
(72) Erfinder: Wandel, Waldemar, 72127 Kusterdingen (DE)
(74) Vertreter: Rüger, Barthelt & Abel

(56) Entgegenhaltungen:
- EP-A1- 2 491 968
- EP-A1- 2 711 545
- WO-A1-01/40042
- WO-A1-2005/034777
- DE-A1-102006 053 609

## Beschreibung

Die Erfindung betrifft eine Pumpeinheit, insbesondere für medizinischen Einsatz, speziell für die Wasserstrahlchirurgie.

Bei der Wasserstrahlchirurgie wird durch ein geeignetes Instrument ein Strahl physiologische Kochsalzlösung auf ein biologisches Gewebe gerichtet, das dadurch vollständig oder teilweise durchtrennt und/oder aufgelöst wird. Damit sind schonende Operationstechniken möglich. Das entsprechende Instrument muss mit dem Behandlungsfluid, insbesondere NaCl-Lösung, mit gewünschtem Druck und/oder gewünschter Fördermenge versorgt werden. Dazu dient im Stand der Technik eine Pumpeinheit, die typischerweise zwei gegenläufig arbeitende separat angetriebene Pumpkolben aufweist. Über entsprechende Ein- und Auslassventile wird sichergestellt, dass die Kolbenpumpen NaCl-Lösung ansaugen und zu dem Instrument hin gerichtet abgeben.

Aus der DE 10 2006 053 609 A1 ist eine medizinische Pumpe für die Wasserstrahlchirurgie bekannt, die zwei Gehäuseteile, einen Ansaugkanal und einen Druckkanal aufweist, die in dem ersten Gehäuseteil ausgebildet sind. In dem zweiten Gehäuseteil sind zwei Pumpzylinder ausgebildet, in denen Pumpkolben angeordnet sind. Von den Zylindern gehen Pumpkanäle ab, wobei in einem der Gehäuseteile Ventilkammerausnehmungen ausgebildet sind. Den Ventilkammerausnehmungen sind Ventilkammerverschlüsse zugeordnet. Die Ventilkammern sind mit Ventilen versehen. Die in den Ventilen vorgesehenen Ventilverschlussglieder sind Kugeln, die durch Federn an Ventilsitze angedrückt sind.

Die Kugeln sind massive Körper, die sowohl für Gase als auch für Flüssigkeiten als undurchlässig gelten müssen.

Solche Pumpeinheiten werden zum Beispiel als Einweggeräte steril bereitgestellt. Bei der Pumpeneinheit handelt es sich um ein medizinisches Sterilprodukt. Dies erfordert, dass die Pumpeinheit unter besonderen Sauberkeitsanforderungen, beispielsweise in einem Reinraum, hergestellt und gegebenenfalls nach der Montage gereinigt wird. Insbesondere muss die Pumpeinheit nach der Montage vollständig sterilisiert werden. Dazu können Gassterilisationsverfahren genutzt werden, bei denen entsprechende Sterilisationsgase, wie beispielsweise Ethylenoxid, Formaldehyd, Per-Essigsäure oder dergleichen, durch die Kanäle der Pumpeinheit geleitet werden. Nachdem die Pumpeinheit aber zum Fördern von Flüssigkeiten, nicht aber zum Fördern von Gasen ausgelegt ist, stellt es eine Herausforderung dar, sicherzustellen, dass das verwendete Sterilisationsgas alle relevanten Stellen der Pumpeinheit wirklich erreicht.

Ein weiteres Problem bei den Pumpeinheiten ergibt sich aufgrund des geforderten großen Bereichs einstellbarer Fördermengen. Sowohl bei schneller als auch insbesondere bei sehr langsamer Kolbenbewegung muss ein gleichmäßiger, nicht pulsierender aussetzungsfreier NaCl-Strahl erzeugt werden. Dies stellt besondere Anforderungen an die Ein- und Auslassventile der Pumpeinheit.

Davon ausgehend ist es Aufgabe der Erfindung, eine verbesserte Pumpeinheit anzugeben.

Diese Aufgabe wird mit der Pumpeinheit nach Anspruch 1 gelöst:
Die erfindungsgemäße Pumpeinheit weist ein Pumpengehäuse auf, das mindestens ein erstes und ein zweites Gehäuseteil aufweist. Vorzugsweise besteht es lediglich aus dem ersten und dem zweiten
   Gehäuseteil. Während eines der Gehäuseteile Pumpzylinder zur Aufnahme von Pumpkolben aufweist, ist das andere Gehäuseteil mit einem Ansaugkanal und einem Druckkanal versehen. Außerdem sind in dem Pumpengehäuse Ventilkammern ausgebildet, in denen Ventilverschlussglieder angeordnet sind. Die Ventilkammern werden durch Ventilkammerausnehmungen und Ventilkammerverschlüsse gebildet. Während eine Ventilkammerausnehmung in einem der Gehäuseteile angeordnet ist, kann der zugeordnete Ventilkammerverschluss an dem anderen Gehäuseteil ausgebildet sein. Damit sind die Ventilkammern, d.h. die Ventilgehäuse von dem Pumpengehäuse selbst gebildet. Zum Beispiel können alle Ventilkammerausnehmungen in dem ersten Gehäuseteil und alle Ventilkammerverschlüsse an dem zweiten Gehäuseteil ausgebildet sein. Es ergibt sich ein einfacher montagefreundlicher Aufbau, bei dem alle Ein- und Auslassventile beim Zusammenfügen der Gehäuseteile komplettiert werden.

Das Ventilverschlussglied ist sterilisationsgaspermeabel ausgebildet. Es besteht beispielsweise aus einem Teller oder Scheibchen aus einem geeigneten Kunststoff, wie beispielsweise Silikon oder einem Elastomer, beispielsweise EPM, EPDM, FPM oder dergleichen. Durch das Zusammenspiel aus geometrischer Form, d.h. dünnwandiger Geometrie des Ventilverschlussglieds, und seiner Materialwahl, können niedermolekulare Sterilisationsgase in ausreichender Rate durch das Ventilverschlussglied hindurchdringen und somit die inneren Kanäle der Pumpeinheit erreichen, ohne dass sie unter Druckanwendung durch die Pumpeinheit gepresst werden müssten. Insbesondere liegt das Ventilverschlussglied ohne oder mit geringer Vorspannung an dem zugeordneten Ventilsitz an. Ventilfedern oder dergleichen sind vorzugsweise nicht vorhanden. Die sich ergebende geringe Flächenpressung an dem Ventilsitz verhindert ein Ankleben des Ventilverschlussglieds am Ventilsitz, ermöglicht insbesondere als Ansaugventil auch das Ansaugen von Flüssigkeiten mit sehr niedriger Flussrate (Strömungsgeschwindigkeit) und fördert die Sterilisation. Es sind nur äußerst geringe Kräfte zum Öffnen des Ventils und somit am Ventil nur äußerst geringe Druckunterschiede zum Öffnen desselben erforderlich.

Vorzugsweise weist die Pumpeinheit ein Ventilverschlussglied auf, das im Querschnitt etwa T-förmig ausgebildet ist, also einen Tellerabschnitt und einen Mittelzapfen aufweist. Der Mittelzapfen kann sich in den Ansaugkanal, den Druckkanal oder den Pumpkanal erstrecken. Insbesondere wenn der Tellerabschnitt des Ventilverschlussglieds bezüglich einer Radialebene asymmetrisch ausgebildet ist, stellt der Mittelzapfen eine wesentliche Montagehilfe dar. Außerdem stellt er beim Zusammenführen der Gehäuseteile sicher, dass die Ventilverschlussglieder richtig positioniert sind und beim Zusammenführen der Gehäuseteile nicht beschädigt werden.

Das Ventilverschlussglied weist vorzugsweise einen steifen Nabenabschnitt, einen relativ steifen Rand und einen dazwischen angeordneten Federabschnitt auf, der den Rand mit dem Nabenabschnitt federnd verbindet. Der Rand weist einen geringeren Durchmesser auf als die Ventilkammerausnehmung, so dass zwischen beiden ein strömungsdurchlässiger Ringspalt ausgebildet ist. Alternativ können in der Wand der Ventilkammerausnehmung Überströmtaschen ausgebildet sein.

Die Federzone ist vorzugsweise eine federnde, durch eine Nut gebildete Ringzone, bei der die in Axialrichtung gemessene Materialdicke des Tellerabschnitts geringer ist, als an anderen Stellen des Tellerabschnitts. Die Nut umgibt den Nabenabschnitt vorzugsweise konzentrisch. Der Nabenabschnitt kann sich so axial federnd gegen den Rand bewegen. Vorzugsweise ist der Tellerabschnitt an der einem Ventilsitz zugewandten Seite konvex gekrümmt. Außerdem weist die ringförmige Federzone vorzugsweise einen Durchmesser auf, der wenigstens so groß ist, wie ein ringförmiger Ventilsitz, der dem Ventilverschlussglied zugeordnet ist.

Die Ventilkammern sind wie beschrieben zwischen der Ventilkammerausnehmung und dem Ventilkammerverschluss ausgebildet. Vorzugsweise sind die Ventilkammerverschlüsse als in die Ventilkammerausnehmung hinein ragende Fortsätze ausgebildet. Die Fortsätze können mit oder ohne Spiel in der Ventilkammerausnehmung sitzen. Bei einer bevorzugten Ausführungsform ist zwischen der zum Beispiel zylindrischen oder leicht konischen Außenumfangsfläche des Ventilkammerverschlusses und der entsprechend gegenüber liegenden Fläche der Ventilkammerausnehmung ein Ringspalt ausgebildet. Dieser ist wenigstens so weit, dass er eine wenigstens geringe radiale Beweglichkeit der Gehäuseteile gegeneinander ermöglicht, bis sie miteinander verbunden sind. Die Beweglichkeit ist so festgelegt, dass die Gehäuseteile durch eine flächenhafte stoffschlüssige Verbindung, insbesondere in einem Reibschweißverfahren, zum Beispiel einem Ultraschallschweißverfahren, miteinander verbunden werden können.

Indem die Ventilkammerverschlüsse an Fortsätzen ausgebildet sind, die die von der Reibschweißnaht festgelegte Ebene überragen, wird die Ventilkammer von dem Reibschweißvorgang, sich eventuell ausbildenden Deformationen oder Materialwülsten nicht betroffen.

Die Ventilverschlussglieder der einzelnen Ventilanordnungen sind vorzugsweise einheitlich, d.h. untereinander gleich ausgebildet. Sie können jedoch auch unterschiedlich ausgebildet sein. Jedes Ventilverschlussglied weist in einem zentralen Bereich einen Dichtabschnitt auf, während an seinem Umfangsrand oder an einer dieser benachbarten Struktur des Pumpengehäuses eine Umströmungsstruktur festgelegt ist. Diese kann eine oder mehrere den Rand des Ventilverschlussglieds durchsetzende Ausnehmungen beinhalten oder durch Ausnehmungen in der benachbarten Anlagefläche des Ventilverschlussglieds ausgebildet sein.

Vorzugsweise ist das Ventilverschlussglied mindestens eines der Ventile an dem Ventilsitz spielfrei anliegend angeordnet. Dabei ist es selbst etwas verformbar, um den von ihm gesteuerten Kanal federnd freigeben zu können. Es können alle Ventile nach dieser Bauart ausgebildet sein. Es ist möglich, alle Ventilverschlussglieder aller Ventile mit gleicher Vorspannung an ihren Ventilsitzen anliegend anzuordnen. Es ist alternativ auch möglich, die Ventilverschlussglieder verschiedener Ventile mit unterschiedlichen Vorspannungen an ihren Ventilsitzen anliegen zu lassen. Z.B. können die Ventilverschlussglieder der Einlassventile mit geringerer Vorspannung oder einer Vorspannung von Null oder mit Spiel an den Ventilsitzen anliegen, währen die Ventilverschlussglieder der Auslassventile mit größerer Vorspannung an ihren Ventilsitzen anliegen.

Weitere Einzelheiten vorteilhafter Ausführungsformen der Erfindung sind Gegenstand der Beschreibung, der Zeichnung oder von Ansprüchen. Es zeigen:
Figur 1 eine erfindungsgemäße Pumpeinheit, in schematisierter Vertikalschnittdarstellung.
Figur 2 ein Einlassventil der Pumpeinheit nach Figur 1, in vergrößerter Vertikalschnittdarstellung.
Figur 3 ein Ventilverschlussglied der Pumpeinheit nach den Figuren 1 und 2.
Figur 4 eine abgewandelte Ausführungsform eines Einlassventils der Pumpeinheit entsprechend Figur 1, in Vertikalschnittdarstellung.
Figur 5 Das Ventilverschlussglied des Einlassventils nach Figur 4, in Perspektivdarstellung und
Figur 6 den zweiten Gehäuseteil der Pumpeinheit nach Figur 1, in vereinfachter Perspektivdarstellung.

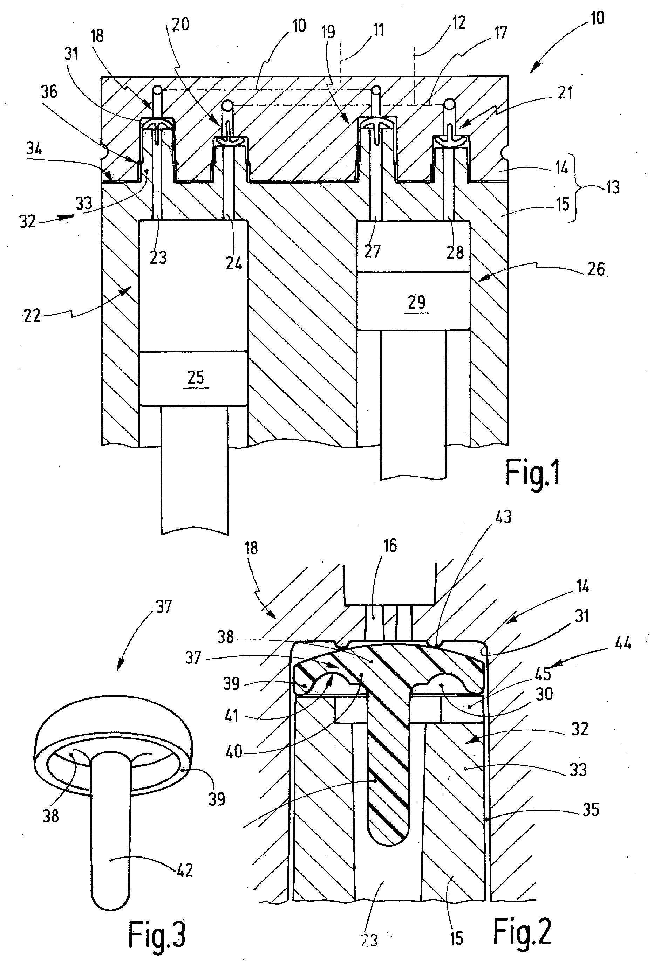

In Figur 1 ist eine Pumpeinheit 10 veranschaulicht, die für den medizinischen Einsatz vorgesehen ist. Sie kann beispielsweise als Pumpe für NaCl-Lösung für die Wasserstrahlchirurgie dienen. Sie saugt an einem in Figur 1 gestrichelt angedeuteten Anschluss 11 NaCl-Lösung oder eine andere gewünschte Flüssigkeit an und fördert diese über einen ebenfalls lediglich schematisch angedeuteten Anschluss 12 zu einem wasserstrahlchirurgischem Instrument.

Die Pumpeinheit 10 weist ein Gehäuse 13 auf, das aus einem ersten Gehäuseteil 14 und einem zweiten Gehäuseteil 15 besteht. Vorzugsweise besteht das Gehäuse 13 lediglich aus diesen beiden Gehäuseteilen, die fest und vorzugsweise unlösbar miteinander verbunden sind. Die Gehäuseteile 14, 15 bestehen vorzugsweise aus einem Kunststoff, vorzugsweise einem ultraschallschweißfähigen Kunststoff.

In dem ersten Gehäuseteil 14 sind ein mit dem Anschluss verbundener Ansaugkanal 16 und ein mit dem Anschluss 12 verbundener Druckkanal 17 ausgebildet. Der Ansaugkanal 16 führt zu Ansaugventilen 18, 19. Der Druckkanal 17 ist mit Auslassventilen 20, 21 verbunden.

Das Einlassventil 18 und das Auslassventil 20 sind dem ersten Pumpzylinder 22 zugeordnet, der mit dem Einlassventil 18 über einen Pumpkanal 23 und mit dem Auslassventil 20 über einen Pumpkanal 24 in Verbindung steht. In dem ersten Pumpzylinder 22 ist ein Pumpkolben 25 angeordnet, der über eine nicht weiter veranschaulichte Kupplung mit einer Antriebseinrichtung verbindbar ist, um in dem Pumpzylinder 22 kontrolliert pumpend hin und her bewegt zu werden.

In dem zweiten Gehäuseteil 15 ist außerdem ein zweiter Pumpzylinder 26 angeordnet, der über Pumpkanäle 27, 28 mit dem Einlassventil 19 und dem Auslassventil 21 verbunden ist. In dem Pumpzylinder 26 ist ein Pumpkolben 29 angeordnet, der über eine nicht weiter veranschaulichte Kupplungseinrichtung mit der Antriebseinrichtung verbindbar ist. Die Antriebseinrichtung sorgt dafür, dass die Pumpkolben 25, 29 gegenläufig so bewegt werden, dass die zu fördernde Flüssigkeit über den Ansaugkanal 16 gleichmäßig angesaugt und über den Druckkanal 17 gleichmäßig abgegeben wird. Die Antriebseinrichtung ist dabei vorzugsweise so ausgebildet, dass sie die Pumpkolben 25, 29 mit variablen Geschwindigkeiten und/oder variablen Hüben gegenläufig bewegen kann, um die Fördermenge und den Förderdruck der geförderten Flüssigkeit in weiten Grenzen wie gewünscht einzustellen.

Der Flüssigkeitszustrom und -abstrom in die und aus den beiden Pumpzylindern 22, 26 steuert die Einlassventile 18, 19 und die Auslassventile 20, 21, die Rückschlagventile sind. Zur weiteren Erläuterung der Beschaffenheit dieser Ventile wird stellvertretend für die übrigen Ventile 19, 20, 21 nachstehend anhand von Figur 2 auf das Einlassventil 18 Bezug genommen.

Zu dem Einlassventil 18 gehört eine Ventilkammer 30, die mit dem Ansaugkanal 16 und dem Pumpkanal 23 in Verbindung steht. Vorzugsweise sind der Ansaugkanal 16 und der Pumpkanal 23 an einander gegenüber liegenden Seiten der Ventilkanal 30 angeordnet.

Zur Ausbildung der Ventilkammer 30 weist eines der Gehäuseteile, hier beispielsweise das erste Gehäuseteil 14, eine Ventilkammerausnehmung 31 auf, der ein an dem jeweils anderen Gehäuseteil, hier dem zweiten Gehäuseteil 15, vorgesehener Ventilkammerverschluss 32 zugeordnet ist. Der Ventilkammerverschluss 32 wird im vorliegenden Ausführungsbeispiel durch einen zapfenartigen Fortsatz 33 gebildet, der ausgehend von einer oberen Stirnfläche 34 des zweiten Gehäuseteils 15 in die Ventilkammerausnehmung 31 ragt. Der Pumpkanal 23 erstreckt sich durch den Fortsatz 33 und mündet in dessen Stirnfläche.

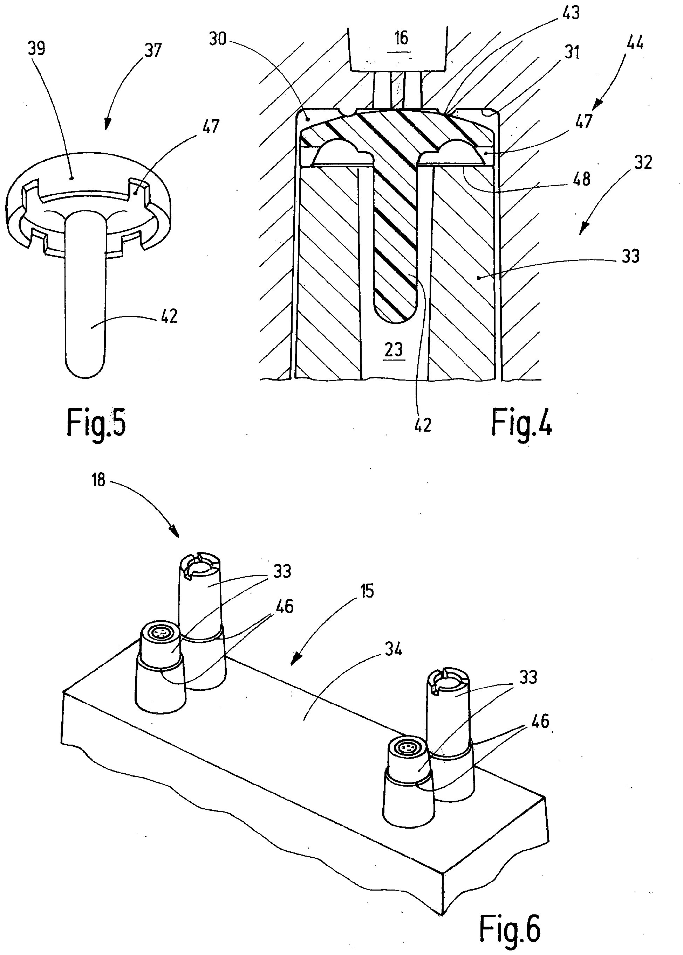

Wie Figur 1 und 6 erkennen lassen, kann der Fortsatz 33 zylindrisch oder auch (ganz leicht) konisch ausgebildet sein. Seine etwa zylindrische Außenumfangsfläche kann an der Innenwand der Ventilkammerausnehmung 31 anliegen oder mit dieser, wie Figur 2 veranschaulicht, einen Ringspalt 35 festlegen. Die beiden Gehäuseteile 14, 15 sind dicht derart miteinander verbunden, dass die Ventilkammer 30 mit der Ansaugkammer 16 und dem Pumpkanal 23 kommuniziert, ansonsten aber dicht ist. Es ist möglich, die Abdichtung durch Presssitz des Fortsatzes 33 in der Ventilkammerausnehmung 31 herzustellen. Auch kann die Abdichtung durch ein gesondertes Dichtungselement, beispielsweise einen Dichtungsring, bewirkt werden. Vorzugsweise wird die Abdichtung jedoch durch eine stoffschlüssige Verbindung, insbesondere eine Reibschweißnaht 36, insbesondere in Gestalt einer Ultraschallschweißnaht erzeugt, die jeden Fortsatz 33 an seinem gesamten Umfang ringsum umgibt. Dazu kann der Fortsatz 33 in seinem von Null verschiedenen Spalt 35 gestuft ausgebildet sein und ebenso kann die Ventilkammerausnehmung 31 eine Ringschulter aufweisen. Werden die Gehäuseteile 14, 15 zusammengefügt und mit Ultraschall beaufschlagt, gewährt der Ringspalt 35 ausreichend Querbeweglichkeit, um zwischen der Stufe und der Ringschulter die Ultraschall- bzw. Reibschweißnaht 36 auszubilden. Diese ist bei allen Ausführungsformen vorzugsweise von der Ventilkammer 30 beabstandet angeordnet.

In der Ventilkammer 30 ist ein Ventilverschlussglied 37 angeordnet. Dieses ist in Figur 3 gesondert veranschaulicht. Das Ventilverschlussglied 37 besteht vorzugsweise vollständig aus Kunststoff, weiter vorzugsweise aus einem einzigen Kunststoffmaterial, das flexibel und für Sterilisationsgase permeabel ist. Beispielsweise kann das Ventilverschlussglied 37 aus EPM, EPDM, FPM oder dergleichen bestehen. Es weist vorzugsweise einen Tellerabschnitt 38 auf, der vorzugsweise eine leicht konvex gewölbt ausgebildete Dichtfläche aufweist und an seinem Umfang mit einem axial vorstehenden ringförmigen Rand 39 versehen ist. Zwischen dem Rand 39 und einem zentralen Nabenabschnitt 40 weist der Tellerabschnitt 38 vorzugsweise eine ringförmige Federzone 41 auf, die durch eine von der konvex gewölbten Seite abgewandte die Nabe 40 umgebende Ringnut gebildet sein kann. Die Federzone 41 ermöglicht ein leichtes Federn des vergleichsweise steiferen Randes 39 gegen den ebenfalls steifen Nabenabschnitt 40 in Axialrichtung. Die federnde Verformung findet vorwiegend in der Federzone 41 statt. Die Federzone wirkt auch als Permeationszone, in der die Wandstärke allenfalls noch wenige Zehntel Millimeter beträgt. Sterilisationsgase können die Federzone 41 in ausreichender Quantität durchdringen, um eine Sterilisation zu bewirken.

Von dem Nabenabschnitt 40 erstreckt sich ein Mittelzapfen 42 vorzugsweise konzentrisch in Axialrichtung weg, wobei der Mittelzapfen 42 in einen der von der Ventilkammer 30 abgehenden Kanäle, beispielsweise in den Pumpkanal 23, ragt, ohne diesen zu versperren. Der Mittelzapfen 42 kann als Orientierungs- und Montagehilfe benutzt werden.

Der konvexen Stirnfläche des Ventilverschlussglieds 37 ist ein beispielsweise durch ein Ringwulst gebildeter Ventilsitz 43 zugeordnet, der den Ansaugkanal 16 umgibt. Der Ventilsitz 43 kann, wie im Beispiel nach Figur 2 dargestellt, durch eine Rundrippe gebildet sein. Diese bewirkt zusammen mit der Nachgiebigkeit des Kunststoffs des Ventilverschlussglieds 37 eine flächenhafte dichtende Anlage zwischen Ventilverschlussglied 37 und Ventilsitz 43. Vorzugsweise ist der Durchmesser des Ventilsitzes 43 höchstens so groß wie der Durchmesser der Federzone 41. Außerdem sind beide vorzugsweise zueinander konzentrisch angeordnet.

Dem Ventilverschlussglied 37 ist eine Überströmstruktur 44 zugeordnet. Diese ist in der Ventilkammer 30 vorzugsweise an der dem Ventilsitz 43 gegenüber liegenden Seite angeordnet. Die Überströmstruktur 44 kann, wie Figur 2 andeutet, durch Radialnuten 45 gebildet sein, von denen sich eine oder mehrere ausgehend von dem Pumpkanal 23 radial nach außen erstrecken. Der Durchmesser des Rands 39 ist geringer als der Durchmesser der Ventilkammer 30. Damit kann der Tellerabschnitt 38 axial von Flüssigkeit umströmt werden.

Die insoweit beschriebene Pumpeinheit 10 arbeitet wie folgt:
In Betrieb werden die Pumpkolben 25, 29 abwechselnd hin- und hergehend bewegt. Der jeweils in Richtung einer Volumenvergrößerung des Pumpzylinders 22 oder 26 laufende Pumpkolben 25 oder 29 saugt dann über den Ansaugkanal 16 Natriumchloridlösung an. Das Ventilverschlussglied 37 wird dabei unter leichter Verformung der Federzone 41 sowie gegebenenfalls weiterer Partien etwa von dem Ventilsitz 43 weg bewegt, um den Durchgang freizugeben.

Bei abgewandelten Ausführungsformen können die Ventilkammer 30 und das Ventilverschlussglied 37 auch so aufeinander abgestimmt sein, dass das Ventilverschlussglied 37 etwas Axialspiel hat. Auch in diesem Fall führt die Saugbewegung des jeweiligen Pumpkolbens 25 oder 29 zum Öffnen des betreffenden Einlassventils 18 oder 19.

Läuft hingegen einer der Pumpkolben 25, 20 in Richtung Volumenverkleinerung des Pumpzylinders 22, 26, bewirkt der Flüssigkeitsdruck und -Strom ein Schließen des jeweiligen Einlassventils 18, 19 und ein Öffnen des jeweiligen Auslassventils 20, 21, das entsprechend den Einlassventilen 18, 19 aufgebaut ist. Der bauliche Unterschied besteht lediglich darin, dass der Ventilsitz 43 nicht auf Seiten des ersten Gehäuseteils 14, sondern auf Seiten des zweiten Gehäuseteils 15 angeordnet ist, um den Pumpkanal 24 bzw. 28 zu umgeben. Entsprechend ist die Überströmstruktur 44 dem Gehäuseteil 14 zugeordnet und am Boden der Ventilkammerausnehmung 31 angeordnet.

Die insoweit beschriebene Pumpeinheit 10 wird wie folgt hergestellt:
Es werden zunächst die Gehäuseteile 14, 15 bereitgestellt und dann auf das in Figur 6 veranschaulichte Gehäuseteil 15 die Ventilverschlussglieder 34 aufgesetzt. Die Ventilverschlussglieder 37 werden dabei auf die entsprechenden Fortsätze 33 jeweils in der von Figur 1 vorgegebenen Orientierung aufgelegt. Danach wird das erste Gehäuseteil 14 mit seinen Ventilkammerausnehmungen 31 auf die Fortsätze 33 aufgesetzt. Es stößt dabei an den an den Fortsätzen 33 ausgebildeten Ringschultern 46 an, bevor seine Unterseite die obere Stirnfläche 34 erreicht hat. Durch Ultraschalleinwirkung bilden sich nunmehr an den Schultern 46 ringförmige Ultraschallschweißnähte bzw. Reibschweißnähte 36 (Figur 1) .

Nach erfolgter Montage wird die Pumpeinheit 10 mit oder ohne Pumpkolben 25, 29 sterilisiert. Dazu wird vorzugsweise das Prinzip der Gassterilisation angewendet, bei der die vorzugsweise aus Kunststoff bestehenden Gehäuseteile 14, 15 keiner oder keiner nennenswerten Temperaturbelastung ausgesetzt werden. Beim Spülen der Pumpeinheit 10 mit Sterilisationsgas wird dieses zum Beispiel über die Anschlüsse 11, 12 in den Ansaugkanal 16 und/oder dem Druckkanal 17 gegeben. Es kann dabei die Einlassventile 18, 19 und/oder die Auslassventile 20, 21 leicht öffnen und/oder durch Diffusion die dünne Kunststoffmembran insbesondere im Bereich des Nabenabschnitts 41 durchwandern. Damit ist eine sichere Sterilisation der Pumpeinheit 10 möglich.

An der beschriebenen Pumpeinheit 10 sind zahlreiche Abwandlungen möglich. Beispielsweise kann die Überströmstruktur 44, wie in Figur 4 und 5 veranschaulicht, durch Ausnehmungen 47 gebildet sein, die in dem Rand 39 des Ventilverschlussglieds 37 ausgebildet sind. Hingegen kann bei dieser Ausführungsform dem Rand 39 eine ebene geschlossene Anlagefläche 48 zugeordnet sein, die die Stirnfläche des Fortsatzes 33 bzw. des Ventilkammerverschlusses 32 bildet. Weiter wird darauf hingewiesen, dass der Kanal 16 in der Nähe des Ventilsitzes 43, wie dargestellt, durch mehrere Einzelkanäle oder auch durch einen einzigen Kanal größeren Querschnitts gebildet sein kann. Weitere Abwandlungen sind möglich.

Die erfindungsgemäße Pumpeinheit 10 weist einen besonders einfachen Aufbau auf, zu dem lediglich zwei Gehäuseteile 14, 15 sowie vier Ventilverschlussglieder 37 und Pumpkolben 25, 29 gehören. Die Ventilverschlussglieder 37 sind untereinander vorzugsweise gleich aufgebaut und in einer Tasche zwischen beiden Gehäuseteilen 14, 15 gefangen. Die Gehäuseteile 14, 15 sind vorzugsweise mittels einer Ultraschallschweißnaht dauerhaft miteinander verbunden. Die Ventilverschlussglieder werden durch scheiben- oder tellerartige in sich federnde Kunststoffteile gebildet, die optional einen Mittelzapfen 42 als Montage- und Orientierungshilfe aufweisen können. Je nach Wunsch, können die Ventilverschlussglieder mit oder ohne Vorspannung an ihrem jeweiligen Ventilsitz 43 anliegen und besonders sicher öffnende und schließende, auf geringste Strömungsgeschwindigkeiten ansprechende leicht sterilisierbare Ventile bilden.

### Bezugszeichenliste:

- 10: Pumpeinheit
- 11: Anschluss zum NaCl-Vorrat
- 12: Anschluss zum wasserstrahlchirurgischen Instrument
- 13: Gehäuse
- 14: erstes Gehäuseteil
- 15: zweites Gehäuseteil
- 16: Ansaugkanal
- 17: Druckkanal
- 18: Ansaugventil
- 19: Ansaugventil
- 20: Auslassventil
- 21: Auslassventil
- 22: erster Pumpzylinder
- 23, 24: Pumpkanäle des ersten Pumpzylinders 22
- 25: Pumpkolben
- 26: zweiter Pumpzylinder
- 27, 28: Pumpkanäle
- 29: Pumpkolben
- 30: Ventilkammer
- 31: Ventilkammerausnehmung
- 32: Ventilkammerverschluss
- 33: Fortsatz
- 34: obere Stirnfläche des zweiten Gehäuseteils 15
- 35: Ringspalt
- 36: Reibschweißnaht
- 37: Ventilverschlussglied
- 38: Tellerabschnitt
- 39: Rand
- 40: Nabenabschnitt
- 41: Federzone
- 42: Mittelzapfen
- 43: Ventilsitz
- 44: Überströmstruktur
- 45: Radialnuten
- 46: Ringschulter
- 47: Ausnehmung
- 48: Anlagefläche

## Patentansprüche

1. Pumpeinheit (10), insbesondere für medizintechnischen Einsatz, insbesondere für die Wasserstrahlchirurgie,
mit einem Pumpengehäuse (13), das ein erstes Gehäuseteil (14) und ein zweites Gehäuseteil (15) aufweist,
mit einem Ansaugkanal (16) und mit einem Druckkanal (17), die in dem ersten Gehäuseteil (14) ausgebildet sind,
mit wenigstens zwei Pumpzylindern (22, 26), die an dem zweiten Gehäuseteil (15) ausgebildet und zur Aufnahme von Pumpkolben (25, 29) eingerichtet sind und von denen je zwei Pumpkanäle (23, 24; 27,28) abgehen,
mit Ventilkammerausnehmungen (31), die in einem der Gehäuseteile (14, 15) ausgebildet sind,
mit Ventilkammerverschlüssen (32), die den Ventilkammerausnehmungen (31) zugeordnet sind, um diese unter Ausbildung einer Ventilkammer (30) abzuschließen, und in denen der Druckkanal (17) oder der Ansaugkanal (16) mündet, **dadurch gekennzeichnet, dass** der Ansaugkanal (16), der Druckkanal (17) und Pumpkanäle (23, 24, 27, 28) in der Ventilkammer (30) mündend angeordnet sind,
mit wenigstens einem Ventilverschlussglied (37), das sterilisationsgasdurchlässig ausgebildet ist.

2. Pumpeinheit nach Anspruch 1, **dadurch gekennzeichnet, dass** das Ventilverschlussglied (37) einen Tellerabschnitt (38) und einen Mittelzapfen (42) aufweist und in einer der Ventilkammern (30) so angeordnet ist, dass sich der Mittelzapfen (42) in einen von dem Ansaugkanal (16), dem Druckkanal (17) und den Pumpkanälen (23, 24, 27, 28) erstreckt.

3. Pumpeinheit nach einem der vorstehenden Ansprüche, **dadurch gekennzeichnet, dass** die Ventilkammerverschlüsse (32) als in die Ventilkammerausnehmungen (31) hinein ragende Fortsätze (42) ausgebildet sind.

4. Pumpeinheit nach einem der vorstehenden Ansprüche, **dadurch gekennzeichnet, dass** die Ventilkammerverschlüsse (32) mit den Ventilkammerausnehmungen (31) einen Ringspalt (35) festlegend ausgebildet sind.

5. Pumpeinheit nach einem der vorstehenden Ansprüche, **dadurch gekennzeichnet, dass** die Gehäuseteile (14, 15) durch eine flächenhafte stoffschlüssige Verbindung (36) miteinander verbunden sind.

6. Pumpeinheit nach Anspruch 5, **dadurch gekennzeichnet, dass** die Verbindung (36) eine Reibschweißnaht ist.

7. Pumpeinheit nach Anspruch 5 oder 6, **dadurch gekennzeichnet, dass** die Verbindung (36) in einer von den Fortsätzen (33) überragten Ebene angeordnet ist.

8. Pumpeinheit nach einem der vorstehenden Ansprüche, **dadurch gekennzeichnet, dass** jeder Ventilkammerverschluss (32) einen Pumpkanal (23, 24, 27, 28) aufweist.

9. Pumpeinheit nach einem der vorstehenden Ansprüche, **dadurch gekennzeichnet, dass** jedem Pumpzylinder (22, 26) mindestens zwei Ventilkammerverschlüsse (32) zugeordnet sind, von denen einer einen als Einlasskanal dienenden Kanal (23, 27) und von denen ein anderer einen als Auslasskanal dienenden Kanal (24, 29) aufweist.

10. Pumpeinheit nach Anspruch 9, **dadurch gekennzeichnet, dass** der den Einlasskanal enthaltende Ventilkammerverschluss (32) mit einem Rand (39) des Ventilverschlussgliedes (37) eine Umströmungsstruktur (44) festlegend ausgebildet ist.

11. Pumpeinheit nach Anspruch 10, **dadurch gekennzeichnet, dass** der Mittelzapfen (42) des Ventilverschlussgliedes (37) in den Ansaugkanal (16) oder in den als Einlasskanal dienenden Pumpkanal (23, 27) ragend angeordnet ist.

12. Pumpeinheit nach einem der vorstehenden Ansprüche, **dadurch gekennzeichnet, dass** der Ansaugkanal (16) an einem Ventilsitz (43) mündet, an dem das Ventilverschlussglied (37) spielfrei anliegt.

13. Pumpeinheit nach Anspruch 9, **dadurch gekennzeichnet, dass** an Pumpkanal (24, 28), der zu dem Druckkanal (17) führt, ein Ventilsitz (43) ausgebildet ist.

14. Pumpeinheit nach Anspruch 13, **dadurch gekennzeichnet, dass** das Ventilverschlussglied (37) an dem Ventilsitz (43) spielfrei anliegend angeordnet ist.

15. Pumpeinheit nach einem der vorstehenden Ansprüche, **dadurch gekennzeichnet, dass** in den Ventilkammern (30) gleiche Ventilverschlussglieder (37) angeordnet sind.

## Claims

1. Pump unit (10), in particular for use in medical technology, in particular for water jet surgery,
with a pump housing (13) which has a first housing part (14) and a second housing part (15),
with an intake channel (16) and with a pressure channel (17) which are formed in the first housing part (14),
with at least two pump cylinders (22, 26) which are formed on the second housing part (15) and are configured to receive pump pistons (25, 29), and from each of which two pump channels depart (23, 24; 27, 28),
with valve chamber recesses (31) which are formed in one of the housing parts (14, 15),
with valve chamber closures (32) which are assigned to the valve chamber recesses (31) in order to close these, forming a valve chamber (30), and into which the pressure channel (17) or intake channel (18) opens,
**characterized in that**
the intake channel (16), the pressure channel (17) and the pump channels (23, 24, 27, 28) are arranged opening into the valve chamber (30),
with at least one valve closing member (37) which is configured so as to be permeable to sterilisation gas.

2. Pump unit according to claim 1, **characterized in that** the valve closing member (37) has a plate portion (38) and a centre peg (42), and is arranged in one of the valve chambers (30) such that the centre peg (42) extends into one of the intake channel (16), pressure channel (17) and pump channels (23, 24, 27, 28).

3. Pump unit according to any of the preceding claims, **characterized in that** the valve chamber closures (32) are formed as extensions (42) protruding into the valve chamber recesses (31).

4. Pump unit according to any of the preceding claims, **characterized in that** the valve chamber closures (32) are formed so as to establish a ring gap (35) with the valve chamber recesses (31).

5. Pump unit according to any of the preceding claims, **characterized in that** the housing parts (14, 15) are connected together by a superficial substance-bonded connection (36).

6. Pump unit according to claim 5, **characterized in that** the connection (36) is a friction weld seam.

7. Pump unit according to claim 5 or 6, **characterized in that** the connection (36) is arranged in a plane over which the extensions (33) project.

8. Pump unit according to any of the preceding claims, **characterized in that** each valve chamber closure (32) comprises a pump channel (23, 24, 27, 28).

9. Pump unit according to any of the preceding claims, **characterized in that** at least two valve chamber closures (32) are assigned to each pump cylinder (22, 26), of which one closure has a channel (23, 27) serving as an inlet channel and another has a channel (24, 29) serving as an outlet channel.

10. Pump unit according to claim 9, **characterized in that** the valve chamber closure (32) containing the inlet channel is formed so as to establish, with an edge (39) of the valve closing member (37), a flow-around structure (44).

11. Pump unit according to claim 10, **characterized in that** the centre peg (42) of the valve closing member (37) is arranged protruding into the intake channel (16) or into the pump channel (23, 27) serving as the inlet channel.

12. Pump unit according to any of the preceding claims, **characterized in that** the intake channel (16) opens at a valve seat (43) on which the valve closing element (37) lies play-free.

13. Pump unit according to claim 9, **characterized in that** a valve seat (43) is formed at the pump channel (24, 28) leading to the pressure channel (17).

14. Pump unit according to claim 13, **characterized in that** the pump closing member (37) is arranged lying play-free on the valve seat (43).

15. Pump unit according to any of the preceding claims, **characterized in that** identical valve closing members (37) are arranged in the valve chambers (30).

## Revendications

1. Unité de pompe (10), en particulier pour une utilisation technique médicale, notamment pour la chirurgie à jet d'eau,
comprenant un corps de pompe (13) qui présente une première partie de corps (14) et une deuxième partie de corps (15),
comprenant un conduit d'aspiration (16) et un conduit de refoulement (17) qui sont réalisés dans la première partie de corps (14),
comprenant au moins deux cylindres de pompe (22, 26) qui sont réalisés sur la deuxième partie de corps (15) et sont conçus pour la réception de pistons de pompe (25, 29) et d'où partent respectivement deux conduits de pompe (23, 24 ; 27, 28),
comprenant des évidements de chambre de soupape (31) qui sont réalisés dans une des parties de corps (14, 15),
comprenant des fermetures de chambre de soupape (32) qui sont associées aux évidements de chambre de soupape (31) pour fermer ceux-ci en formant une chambre de soupape (30) et dans lesquelles débouche le conduit de refoulement (17) ou le conduit d'aspiration (16), **caractérisée en ce que**
le conduit d'aspiration (16), le conduit de refoulement (17) et des conduits de pompe (23, 24, 27, 28) sont disposés de façon à déboucher dans la chambre de soupape (30),
avec au moins un élément d'obturation de soupape (37) qui est réalisé de manière à laisser passer un gaz de stérilisation.

2. Unité de pompe selon la revendication 1, **caractérisée en ce que** l'élément d'obturation de soupape (37) présente une partie formant tête (38) et une tige centrale (42) et est disposé dans l'une des chambres de soupape (30) de manière à ce que la tige centrale (42) s'étende dans l'un des conduits parmi le conduit d'aspiration (16), le conduit de refoulement (17) et les conduits de pompe (23, 24, 27, 28).

3. Unité de pompe selon l'une des revendications précédentes, **caractérisée en ce que** les fermetures de chambre de soupape (32) sont réalisées sous forme de saillies (42) avançant dans les évidements de chambre de soupape (31).

4. Unité de pompe selon l'une des revendications précédentes, **caractérisée en ce que** les fermetures de chambre de soupape (32) sont réalisées de manière à définir un espace annulaire (35) avec les évidements de chambre de soupape (31).

5. Unité de pompe selon l'une des revendications précédentes, **caractérisée en ce que** les parties de corps (14, 15) sont reliées l'une à l'autre par une liaison par matière (36) plane.

6. Unité de pompe selon la revendication 5, **caractérisée en ce que** la liaison (36) est un joint de soudure par friction.

7. Unité de pompe selon la revendication 5 ou 6, **caractérisée en ce que** la liaison (36) est réalisée dans un plan par rapport auquel les saillies (33) dépassent.

8. Unité de pompe selon l'une des revendications précédentes, **caractérisée en ce que** chaque fermeture de chambre de soupape (32) présente un conduit de pompe (23, 24, 27, 28).

9. Unité de pompe selon l'une des revendications précédentes, **caractérisée en ce que** sont associées à chaque cylindre de pompe (22, 26), au moins deux fermetures de chambre de soupape (32) dont une présente un conduit (23, 27) servant de conduit d'admission et dont une autre présente un conduit (24, 29) servant de conduit de sortie.

10. Unité de pompe selon la revendication 9, **caractérisée en ce que** la fermeture de chambre de soupape (32) comportant le conduit d'admission est réalisée de façon à définir une structure d'écoulement de contournement (44), conjointement avec un bord (39) de l'élément d'obturation de soupape (37).

11. Unité de pompe selon la revendication 10, **caractérisée en ce que** la tige centrale (42) de l'élément d'obturation de soupape (37) est disposée de manière à avancer dans le conduit d'aspiration (16) ou dans le conduit de pompe (23, 27) servant de conduit d'admission.

12. Unité de pompe selon l'une des revendications précédentes, **caractérisée en ce que** le conduit d'aspiration (16) débouche sur un siège de soupape (43) contre lequel l'élément d'obturation de soupape (37) est en appui sans jeu.

13. Unité de pompe selon la revendication 9, **caractérisée en ce qu'**un siège de soupape (43) est réalisé sur le conduit de pompe (24, 28) qui mène au conduit de refoulement (17).

14. Unité de pompe selon la revendication 13, **caractérisée en ce que** l'élément d'obturation de soupape (37) est disposé de manière à être en appui sans jeu contre le siège de soupape (43).

15. Unité de pompe selon l'une des revendications précédentes, **caractérisée en ce que** des éléments d'obturation de soupape (37) identiques sont disposés dans les chambres de soupape (30).
